(19) Europäisches Patentamt
European Patent Office
Office européen des brevets

(11) EP 3 364 964 B1

(12) **FASCICULE DE BREVET EUROPEEN**

(45) Date de publication et mention de la délivrance du brevet:
**26.08.2020 Bulletin 2020/35**

(21) Numéro de dépôt: **16806279.2**

(22) Date de dépôt: **25.10.2016**

(51) Int Cl.:
*A61K 31/315* (2006.01) *A61K 31/352* (2006.01)
*A61P 17/10* (2006.01) *A61K 33/30* (2006.01)

(86) Numéro de dépôt international:
**PCT/IB2016/001634**

(87) Numéro de publication internationale:
**WO 2017/068424 (27.04.2017 Gazette 2017/17)**

(54) **COMPOSITION A BASE DE DIHYDROMYRICETINE ET D'UN SEL DE ZINC POUR LE TRAITEMENT DE L'ACNE ET DES PEAUX GRASSES**

ZUSAMMENSETZUNG AUF BASIS VON DIHYDROMYRICETIN UND EINEM ZINKSALZ ZUR BEHANDLUNG VON AKNE UND FETTIGER HAUT

COMPOSITION BASED ON DIHYDROMYRICETIN AND A ZINC SALT FOR THE TREATMENT OF ACNE AND OILY SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.10.2015 FR 1560091**

(43) Date de publication de la demande:
**29.08.2018 Bulletin 2018/35**

(73) Titulaire: **Thorel, Jean-Noël**
**75014 Paris (FR)**

(72) Inventeur: **Thorel, Jean-Noël**
**75014 Paris (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A2- 1 484 086 JP-A- 2000 136 131**

- **Anonymous: "8 Days AntiDouble Chin Serum for Women", Internet , avril 2010 (2010-04), XP002757529, Extrait de l'Internet: URL:www.gnpd.com [extrait le 2016-05-06]**
- **Anonymous: "Bioderma Sébium AI", Internet , 28 décembre 2014 (2014-12-28), XP002757530, Extrait de l'Internet: URL:www.bioderma.com [extrait le 2016-05-06]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### Domaine de l'invention

La présente demande a pour objet une composition cosmétique et/ou dermatologique comprenant de la dihydromyricétine (DHM), dont l'intérêt dans la prévention et le traitement de l'acné est rapporté dans la présente divulgation. De manière avantageuse, la DHM est combinée avec un sel de zinc tel que le gluconate de zinc. L'association de ces deux actifs s'avère très prometteuse pour le traitement de l'acné ou plus généralement des peaux grasses.

### Etat de la technique

L'acné est une pathologie inflammatoire affectant le follicule pilo-sébacé, touchant le visage, le thorax et le dos. C'est une maladie extrêmement fréquente qui atteint plus de 80% des adolescents. Chez environ 50% des individus affectés par l'acné juvénile, l'acné persiste à l'âge adulte. Dans la plupart des cas, l'acné n'est pas une pathologie aiguë mais une condition chronique, qui nécessite par conséquent des traitements prolongés. Dans cette perspective, il est impératif que les traitements administrés aient une toxicité minimale.

D'un point de vue clinique, l'acné est par définition une affection polymorphe qui associe des lésions rétentionnelles (microkystes, comédons) et/ou des lésions inflammatoires (papules, pustules ou nodules). Ces deux types de lésions peuvent coexister ou se succéder lors de poussées.

L'étiopathogénie de l'acné est multifactorielle et reste en partie à élucider. Il a cependant été établi que la quantité et la qualité du sébum jouent un rôle clé.

Le sébum est un produit de sécrétion lipidique, riche en acides gras et notamment en squalène, un carbure aliphatique à 30 atomes de carbone, précurseur du cholestérol. Le sébum est produit par des cellules dédiées, appelées sébocytes. Dans des conditions physiologiques, il joue un rôle positif notamment dans la protection de la peau et dans la formation du film hydrolipidique. Toutefois, des modifications quantitatives ou qualitatives du sébum peuvent favoriser l'acné. Ainsi, l'hyperproduction de sébum et/ou l'altération de la composition des lipides du sébum, notamment leur oxydation, contribuent à l'apparition et à la sévérité de l'acné.

La production excessive de sébum par les glandes sébacées, également appelée hyperséborrhée, se manifeste au niveau du cuir chevelu et du visage, surtout sur la région médiofaciale, mais aussi sur la partie supérieure du dos et de la poitrine. Ce sont d'ailleurs les zones les plus touchées par l'acné. La peau hyperséborrhéique a un aspect luisant, condition généralement appelée « peau grasse ».

L'hyperséborrhée est particulièrement prononcée lors de la puberté, quand l'augmentation du taux des hormones circulantes (en particulier de la testostérone) cause une hyperactivation des glandes, directement corrélée avec les poussées d'acné caractéristiques de l'adolescence. L'hormone IGF-1 en particulier semble être directement responsable de l'apparition des symptômes cutanés de l'acné. Ainsi, une corrélation entre sévérité de l'acné et taux sérique d'IGF-1 a été établie (Cappel *et al.* 2005). En outre, l'IGF-1 et son récepteur sont surexprimés dans les lésions d'acné (Isard *et al.* 2011). Il a aussi été montré que IGF-1 stimule la synthèse des lipides dans les glandes sébacées (Deplewski *et al.* 2000) et induit l'activité de la 5$\alpha$ réductase (Horton *et al.* 1993), une enzyme impliquée de la production du sébum. De plus, de façon cohérente avec l'implication de cette hormone dans l'apparition de l'acné, un pic de synthèse d'IGF-1 est observé au cours de l'adolescence (Deplewski *et al.* 1999).

La production excessive de sébum crée un environnement favorable à la prolifération bactérienne, en particulier de la bactérie Gram positif anaérobie *Propionibacterium acnes* (*P.acnes*), ce qui aggrave la pathologie en accentuant l'état inflammatoire cutané. Un traitement efficace limitant cette prolifération est le peroxyde de benzoyle, un agent antimicrobien à large spectre. Le peroxyde de benzoyle présente toutefois de nombreux effets indésirables, notamment un effet irritant, la génération de radicaux libres, un effet photosensibilisant, ainsi que la décoloration des vêtements. Par ailleurs, plusieurs antibiotiques de la famille de la tétracycline sont administrés par la voie topique ou orale afin de contrôler la prolifération de *P. acnes* et de ce fait, traiter l'acné. Les tétracyclines, qui possèdent également des propriétés anti-inflammatoires, agissent efficacement et rapidement sur l'acné. Cependant, l'utilisation prolongée d'antibiotiques est de plus en plus décriée car elle entraine l'apparition de résistance aux antibiotiques chez les bactéries pathogènes. De surcroit, les effets d'antibiotiques sur l'ensemble du microbiome humain et ses équilibres sont encore mal connus. Les sels de zinc présentent également un intérêt dans le traitement de l'acné en raison de leur activité antimicrobienne, mais aussi anti-inflammatoire et d'inhibition de l'activité de la 5$\alpha$ réductase (Stamatiadis *et al.* 1988). Toutefois, l'efficacité des sels de zinc en tant qu'actifs à utiliser seuls dans le traitement de l'acné reste limitée.

Un traitement prometteur contre l'acné vise donc la réduction de l'hyperséborrhée. Dans ce cadre, la prise orale d'isotrétinoïne (un dérivé de la vitamine A) a montré de bons résultats. Le mécanisme d'action systémique de l'isotrétinoïne semble reposer principalement sur une modification des glandes sébacées et une suppression de leur activité (Orfanos *et al.* 1998). Ainsi, les traitements à base d'isotrétinoïne permettent une atténuation significative, voire la disparition parfois permanente des symptômes cutanés de l'acné. Toutefois, la prise de fortes doses d'isotrétinoïne

par voie orale est associée à de nombreux effets secondaires indésirables, notamment tératogènes. Pour ces raisons, l'isotrétinoïne est désormais réservée au traitement dermatologique de l'acné récalcitrante nodulaire grave, et strictement contre-indiquée pendant la grossesse.

Il subsiste donc un besoin évident de nouveaux actifs qui puissent diminuer l'hyperséborrhée sans entrainer d'effets secondaires.

Comme déjà dit, en plus des effets délétères de l'excès de sébum, des études ont montré que la composition du sébum joue un rôle actif dans l'acné. En particulier, il a été observé que chez les patients acnéiques, la quantité de squalène est augmentée dans le sébum, où il est en outre retrouvé sous forme oxydée. Ainsi, le sébum des patients atteints d'acné est particulièrement riche en squalène oxydé. Or, le squalène oxydé est à l'origine de l'épaississement du sébum qui cause l'obstruction des pores, mais aussi une hyperkératinisation au niveau des follicules pilo-sébacés, l'hyperprolifération de la bactérie *P. acnes* et l'induction d'une réaction inflammatoire locale responsable du développement des lésions d'acné (Saint Léger *et al.* 1986 ; Chiba *et al.* 1999 ; Chiba *et al.* 2000; Ottaviani *et al.* 2006).

Pour limiter les effets potentiellement néfastes du squalène oxydé présent dans le sébum, l'organisme adopte une stratégie d'auto-défense en libérant de la vitamine E à la surface de la peau. Or, pour des raisons encore inconnues, le niveau cutané de vitamine E est réduit chez les patients acnéiques, ainsi incapable de jouer son rôle protecteur (Thiele *et al.* 1999 ; Picardo *et al.*, 2009 ; Ottaviani *et al.*, 2010).

Selon cet aspect, il est donc souhaitable de limiter l'oxydation du squalène présent dans le sébum. Des compositions cosmétiques ou dermatologiques à usage topique présentant une telle activité sont par exemple décrites dans le document EP 2 583 662.

Outre l'aspect inesthétique des poussées d'acné, les lésions d'acné donnent souvent lieu à la formation de cicatrices, qui représentent la première crainte des patients acnéiques.

En pratique, il existe trois types de cicatrices d'acné. Les cicatrices maculeuses érythémateuses ou pigmentées se présentent comme des taches rouges ou foncées. Elles ne se sont pas permanentes et tendent à disparaître rapidement. Les cicatrices hypertrophiques ou chéloïdiennes sont caractérisées par une hyperprolifération post-lésionnelle des tissus et sont donc en relief. La majorité des cicatrices d'acné (entre 80% et 90%) appartiennent au troisième groupe, les cicatrices atrophiques par perte de substance. Ces cicatrices, caractérisées par des dépressions plus ou moins prononcées, peuvent être souples, fibreuses ou semi-rigides. Un type particulier de cicatrices atrophiques sont les cicatrices en « pic à glace », qui sont punctiformes et qui peuvent être très profondes.

La pathogénie des cicatrices d'acné atrophiques n'est pas complètement élucidée. Une corrélation existe entre la durée ou l'ampleur de la réponse inflammatoire, et la formation de cicatrices atrophiques.

Les métalloprotéines matricielles (ou MMP pour « Matrix Metallo-Proteinase ») jouent un rôle clef dans le remodelage post-lésionnel de la matrice extracellulaire. Dans le cas d'une plaie normale de taille moyenne, elles permettent la disparition complète de la cicatrice en quelques jours ou semaines. Ces enzymes sont secrétées par les fibroblastes et les kératinocytes en réponse à la stimulation par plusieurs cytokines (IGF1, EGF, TNF$\alpha$, TGF$\beta$ et PDGF).

On considère désormais que la réponse inflammatoire typique des lésions d'acné cause une hyperactivation des MMPs, telles que les collagénases, les gélatinases et les élastases. En effet, dans le contexte de l'acné, la réponse inflammatoire est exacerbée et prolongée, et cause une augmentation chronique du niveau des cytokines inflammatoires. Cette augmentation induit un déséquilibre entre l'expression des MMP et des inhibiteurs tissulaires des MMP, les TIMP (pour « Tissue Inhibitor of Metalloproteinase »). Dans le cas de cicatrices atrophiques, l'hyperactivité des MMP lytiques, consécutive à l'acné, causerait une diminution dans la déposition des fibres de collagène pendant le processus de régénération et de remodelage de la matrice extracellulaire, conduisant à la formation de dépressions visibles à l'œil nu (Chivot et *al.* 2006).

Il n'existe à ce jour aucun traitement préventif efficace de la formation des cicatrices atrophiques. Au mieux, celles-ci sont traitées après leur formation par des techniques chirurgicales (punch, incision sous-cutanée ou subcision) ou des techniques de relissage (dermabrasion, laser-abrasion, peeling, photothermolyse fractionnée...) (Chivot *et al.* 2006).

Comme il ressort de ce qui précède, l'acné est une pathologie multifactorielle pour laquelle il existe un besoin permanent d'identifier de nouveaux actifs ou de nouvelles combinaisons d'actifs susceptibles d'agir sur l'un ou plusieurs des mécanismes mentionnés ci-dessus.

## Description de l'invention

De façon surprenante, le Demandeur a montré que des compositions comprenant de la dihydromyricétine, avantageusement en combinaison avec d'autres actifs, permettent de répondre à ce besoin, notamment grâce aux propriétés de cette molécule, en particulier sa capacité à diminuer l'hyperproduction de sébum par les sébocytes, à protéger le squalène du sébum contre l'oxydation, à inhiber l'activité des enzymes de type MMP, et à inhiber la croissance de *P. acnes*, propriétés identifiées pour la première fois dans le cadre de la présente demande. Il est par ailleurs rapporté l'avantage d'associer cette molécule à un sel de zinc, tel que le gluconate de zinc. Le Demandeur a également mis en

évidence l'action synergique de l'association de la DHM et un sel de zinc sur la suppression de l'activité des enzymes de type MMP. De telles compositions cosmétiques ou dermatologiques présentent donc un intérêt évident dans la prévention et le traitement de l'acné et de ses symptômes, et plus généralement pour le soin des peaux grasses ou à tendance acnéique.

La dihydromyricétine (DHM), également appelée ampélopsine ou ampéloptine, est un flavanolol retrouvé dans plusieurs extraits végétaux qui sont utilisés dans la médecine traditionnelle chinoise, japonaise et coréenne pour le traitement des fièvres, des affections du foie, et des troubles digestifs. La DHM est notamment utilisée dans des préparations pour la prise en charge de la veisalgie ou « gueule de bois » (Shen *et al.* 2012). Des études menées sur la molécule purifiée ont effectivement montré un effet hépato-protecteur pour la DHM et les préparations médicamenteuses buvables la contenant, comme décrit dans CN1483801. Des études récentes ont par ailleurs montré que la DHM possède des propriétés anticancéreuses (Wu *et al.* 2013). En cosmétique, la DHM est utilisée dans des préparations « anticellulite » ou pour le traitement du double-menton, grâce à ses effets sur le métabolisme et la différenciation des adipocytes. On la retrouve aussi dans des préparations aptes à retarder la repousse des poils. JP2000136131 suggère l'utilisation de la DHM ou ampélopsine C pour le traitement de l'acné au vu de son activité inhibitrice de l'enzyme 5$\alpha$-réductase. Toutefois, l'efficacité de la DHM sur l'acné n'est pas démontrée dans ce document.

Les sels de zinc sont des agents antimicrobiens connus et le gluconate de zinc est utilisé couramment dans le traitement de l'acné, comme décrit dans le document EP 1 484 086 qui divulgue l'utilisation de l'association du gluconate de zinc et gluconate de cuivre pour traiter l'acné.

Toutefois et à la connaissance du Demandeur, les propriétés de la DHM, avantageusement lorsque combinée à d'autres molécules décrites ci-après, de préférence lorsque combinée à des sels de zinc, rapportées dans la présente demande, et les applications qui en découlent, n'ont jamais été démontrées.

Selon un premier aspect, la présente invention concerne une composition cosmétique ou dermatologique comprenant de la DHM, un sel de zinc et un antioxydant lipophile choisi parmi le propyl gallate, l'octyl gallate et le dodécyl gallate.

La DHM, ampélopsine ou ampéloptine [(2R,3R)-3,5,7-trihydroxy-2-(3,4,5-trihydroxyphenyl)-2,3-dihydrochromen-4-one ; numéro CAS 27200-12-0), est un flavonol appartenant au groupe de flavonoïdes ayant la structure suivante :

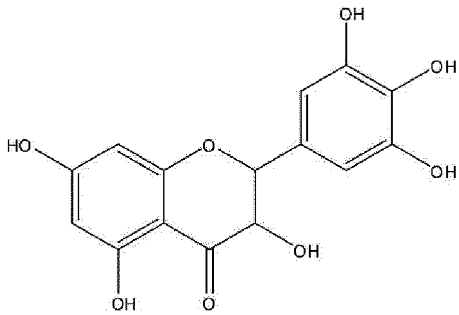

La DHM est l'un des principaux anthocyanidols rencontrés dans la nature, notamment dans les plantes (Gerats *et al.* 1982). La DHM est généralement obtenue à partir de plantes ou d'extraits de plantes. Elle peut également être obtenue à partir de champignons ou de bactéries, ou encore être synthétisée. Des plantes ou extraits de plantes servant de source de DHM sont par exemple les plantes *Myrica cerifera, Ampelopsis spp., Cercidiphyllum japonicum, Hovenia dulcis, Rhododendron cinnabarinum, Pinus spp, Cedrus spp.* et *Salix spp.*

En pratique, la DHM peut être utilisée dans la composition de l'invention sous forme de DHM purifiée ou isolée, de préférence de pureté au moins égale à 60%, 70%, 80%, 90% voire au moins égale à 95%. Alternativement, elle peut se présenter sous la forme d'un extrait de plante comprenant de la DHM, représentant avantageusement de 1% à 70% en poids par rapport au poids total de l'extrait.

Au sens de la présente demande, les pourcentages de pureté des composés indiqués correspondent aux pourcentages en poids par rapport au poids total du composé ajouté.

Selon un mode de réalisation particulier, la DHM est issue d'un extrait de la plante *Myrica cerifera*, notamment des feuilles, fleurs ou écorces. Selon un mode de réalisation préféré, la DHM est isolée et purifiée à partir de la plante *Myrica cerifera*, comme par exemple le produit titré en DHM, commercialisé par la société PROVITAL sous la dénomination TELOCAPIL SPE™ ou MYRICELINE™.

De préférence, la DHM représente typiquement de 0,0001% à 10% en poids de la composition, avantageusement de 0,001% à 2% en poids de la composition, encore plus avantageusement 0,01% en poids de la composition.

Avantageusement, la composition de l'invention comprend en outre au moins un autre ingrédient de préférence choisi parmi :

- la biochanine A, ou un extrait végétal comprenant de la biochanine A, avantageusement un extrait de trèfle rouge;
- un extrait de *Ginkgo biloba,* avantageusement un extrait de *Ginkgo biloba* comprenant des flavonoïdes ;
- un méroterpène, avantageusement du bakuchiol ;
- un polyol, avantageusement choisi parmi le xylitol, le sorbitol et le mannitol ;
- un agent kératolytique, avantageusement choisi parmi l'acide salicylique, l'acide glycolique, l'acide citrique, l'acide malique et l'acide lactique ;
- l'acide glycyrrhétinique ou l'un de ses dérivés ou sels.

Les sels de zinc sont des agents antibactériens connus et peuvent contribuer à limiter la prolifération de *P. acnes*. De façon surprenante, le Demandeur a établi que les sels de zinc stimulent les effets de la DHM. Le Demandeur a notamment déterminé que l'association de DHM et d'au moins un sel de zinc, avantageusement le gluconate de zinc, est particulièrement bénéfique dans le traitement de l'acné. En effet et tel qu'étayé par la partie expérimentale, cette combinaison d'ingrédients possède un effet synergique inhibiteur sur plusieurs phénomènes à l'origine de l'acné, de l'inflammation et des cicatrices qui en découlent. Cet effet synergique est notamment observé sur l'activité des MMPs, les enzymes responsables de la formation de cicatrices atrophiques.

Selon l'invention, le zinc est apporté sous forme de sel de zinc permettant de libérer le zinc sous sa forme ionique $Zn^{2+}$. Plusieurs sels de zinc, seuls ou en combinaison, sont adaptés à l'utilisation dans des compositions selon l'invention. De préférence, le sel de zinc est choisi parmi le gluconate de zinc, le sulfate de zinc et l'oxyde de zinc. Dans un mode de réalisation préféré, le sel de zinc est le gluconate de zinc. Selon un mode de réalisation particulier, le sel de zinc n'est pas un oxyde de zinc, par ailleurs connus pour leur utilisation comme écrans solaires.

De préférence, le sel de zinc représente de 0,1% à 10% en poids de la composition, avantageusement de 1% à 5%, encore plus avantageusement 1%, 2% ou 3% en poids de la composition.

La biochanine A (5,7-dihydroxy-3-(4-methoxyphenyl)-chromen-4-one ; numéro CAS 491-80-5) est un flavonoïde ayant la structure suivante :

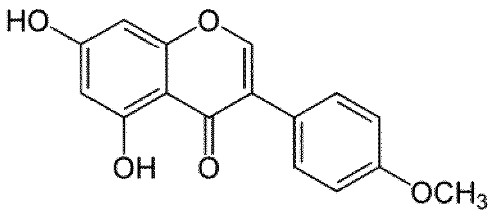

La biochanine A possède des propretés antioxydantes avérées et est utilisée en cosmétique pour protéger les cellules de la peau des méfaits des rayonnements UV. La biochanine A possède également une activité inhibitrice des enzymes 17βHSD (Le Lain *et al.* 2001) et 3βHSD (Ohno *et al.* 2004), deux enzymes impliquées dans la synthèse des androgènes et notamment de la testostérone. Comme déjà dit, la testostérone stimule la production de sébum et l'apparition des symptômes cutanés de l'acné.

La biochanine A est généralement obtenue à partir de plantes ou d'extraits de plantes comprenant cet actif. Des plantes ou extraits de plante servant de source de biochanine A sont par exemple le *Glycine max* (soja), le *Cicer arietinum* (pois chiche) la *Medicago sativa* (luzerne) ou le *Trifolium pratense* (trèfle rouge).

En pratique, la biochanine A peut être apportée par l'ajout de biochanine A purifiée, de préférence ayant un degré de pureté au moins égal à 60%, voire au moins égal à 70%, 80%, 90% voire au moins égal à 95%, ou par l'ajout d'un extrait végétal comprenant de la biochanine A. Des extraits hautement concentrés en biochanine A peuvent être obtenus, à titre d'exemple, en utilisant un mélange de propylène glycol et eau pour extraire la fraction hydrophile des parties aériennes des plantes susmentionnés. Les extraits ainsi obtenus sont par la suite clarifiés et soumis à une filtration stérilisante.

Selon un mode de réalisation particulier, la composition comprend un extrait végétal comprenant de la biochanine A, de préférence à hauteur de 0,1 g/L à 10 g/L de biochanine A par rapport à l'extrait végétal.

Selon un mode de réalisation, l'extrait végétal comprenant de la biochanine A est un extrait de la plante *Trifolium pratense* (trèfle rouge). De préférence, l'extrait végétal comprenant de la biochanine A est un extrait des parties aériennes du trèfle rouge, avantageusement des feuilles. Dans un mode de réalisation particulier, l'extrait comprenant de la biochanine A est un extrait titré correspond à l'INCI *Trifolium pratense* (clover) leaf extract. A titre d'exemple, l'extrait hydroglycolique de feuilles de trèfle rouge commercialisé par la société Greentech peut être utilisé dans le cadre de l'invention.

De préférence, l'extrait comprenant de la biochanine A représente de 0,001% à 1% en poids de la composition, avantageusement de 0,01% à 0,1% en poids de la composition. De manière particulièrement avantageuse, l'extrait comprenant de la biochanine A est un extrait sec.

De façon avantageuse, la composition selon l'invention comprend un extrait de *Ginkgo biloba.*

De manière avantageuse, l'extrait de *Ginkgo biloba* est un extrait de *Ginkgo biloba* comprenant des flavonoïdes, avantageusement titré en flavonoïdes. L'extrait de *Ginkgo biloba* est avantageusement un extrait de feuilles. Dans un mode de réalisation particulier, l'extrait de *Ginkgo biloba* correspond à l'INCI *Ginkgo biloba* leaf extract. A titre d'exemple, l'extrait glycolique de feuilles de *Ginkgo biloba* à 5% en matière sèche commercialisé par la société Greentech peut être utilisé dans le cadre de l'invention.

De préférence, l'extrait de *Ginkgo biloba* représente de 0,0001% à 1% en poids de la composition, avantageusement de 0,001% à 0,1% en poids de la composition.

De façon avantageuse, la composition selon l'invention comprend un méroterpène.

Les méroterpènes ont une action antimicrobienne mais sont également utiles pour réduire l'oxydation du sébum et supprimer l'activité des élastases et collagénases, des enzymes impliquées dans la formation de cicatrices atrophiques d'acné.

Les méroterpènes sont généralement obtenus à partir de plantes ou d'extraits de plantes. Ils peuvent également être obtenus à partir de champignons ou encore être synthétisés. Des plantes servant de source pour les méroterpènes sont par exemple *Psoralea coryfolia, Psoralea spp.* et *Otholobium pubescens.*

En pratique, le méroterpène peut être apporté par l'ajout de méroterpène isolé et purifié, de préférence ayant un degré de pureté au moins égal à 60%, voire au moins égal à 70%, 80%, 90% voire au moins égal à 95%, ou par l'ajout d'un extrait végétal comprenant le méroterpène.

Selon un mode de réalisation préféré, la composition comprend un extrait végétal comprenant le méroterpène, de préférence de 1% à 70% en poids de méroterpène par rapport au poids total de l'extrait. L'extrait végétal comprenant le méroterpène est avantageusement un extrait de plante choisie parmi *Psoralea coryfolia, Psoralea spp.* et *Otholobium pubescens.*

A titre d'exemple, les méroterpènes décrits dans le document WO2008/143761 et WO2008/140673 peuvent être mis en œuvre dans le cadre de la présente invention.

Selon un mode de réalisation préféré, le méroterpène mis en œuvre dans le cadre de l'invention est le bakuchiol (4-[(1E,3S)-3-ethenyl-3,7-dimethylocta-1,6-dienyl]phenol), (numéro CAS : 10309-37-2) ayant la structure suivante :

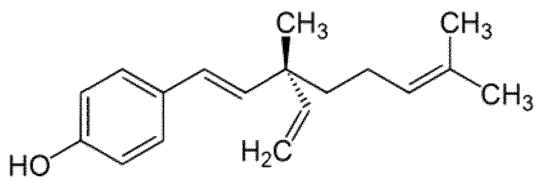

Avantageusement, le bakuchiol est apporté par l'ajout d'un extrait de plante le comprenant, de préférence un extrait de graines de *Psoralea coryfolia.* Selon un autre mode de réalisation, le bakuchiol est isolé et purifié à partir de la plante *P. coryfolia*, avantageusement de ses graines, comme par exemple le produit titré en bakuchiol, commercialisé par la société SYTHEON LTD sous la dénomination Sytenol®. Dans ce produit, le bakuchiol a une pureté supérieure à 95%.

De préférence, le méroterpène représente de 0,01% à 5% en poids de la composition, avantageusement de 0,1% à 2% en poids de la composition, encore plus avantageusement 0,25% en poids de la composition.

La composition selon l'invention peut comprendre un polyol, avantageusement choisi parmi le xylitol, le sorbitol et le mannitol. De préférence, le polyol est le xylitol.

Le xylitol peut être du xylitol en poudre, par exemple d'origine végétale, notamment obtenu à partir du bois par hydrogénation des xyloses, présentant de préférence une pureté supérieure à 95%. Le mannitol peut par exemple être du mannitol en poudre, d'origine végétale (maïs, pomme de terre, blé), de préférence présentant une pureté supérieure à 98%. A titre d'exemple, la société IMCD commercialise le produit Pearlitol® pouvant être mis en œuvre dans le cadre de l'invention.

De manière adaptée, le polyol représente de 0,0001% à 10% en poids de la composition, avantageusement de 0,001% à 2% en poids de la composition, encore plus avantageusement 0,1% en poids de la composition.

De manière avantageuse, la composition selon la présente demande comprend un antioxydant lipophile. A titre d'exemple, les antioxydants lipophiles décrits dans le document FR 2 857 266 peuvent être utilisés dans le cadre de la demande. Dans un mode de réalisation préféré de l'invention, l'antioxydant lipophile est le propyl gallate.

Selon un mode de réalisation, l'antioxydant lipophile représente de 0,001% à 1% en poids de la composition, avantageusement de 0,01% à 0,5% en poids de la composition.

Selon un mode de réalisation préféré, la composition selon l'invention comprend un agent kératolytique, de préférence choisi parmi l'acide salicylique, l'acide glycolique, l'acide citrique, l'acide malique et l'acide lactique, avantageusement l'acide glycolique. De préférence, l'agent kératolytique représente de 1% à 10% en poids de la composition,

avantageusement de 2% à 8% en poids de la composition, encore plus avantageusement 5% en poids de la composition.

Selon un autre mode de réalisation, la composition selon l'invention comprend de l'acide glycyrrhétinique ou l'un de ses dérivés ou sels. L'acide glycyrrhétinique est un agent apaisant connu utile pour le traitement des lésions inflammatoires dans l'acné.

En pratique, l'acide glycyrrhétinique peut être apporté par l'ajout du composé purifié, de préférence de pureté au moins égale à 90%, ou par l'ajout d'un extrait de plante le comprenant ou titré en cet actif. De préférence, l'extrait de plante comprenant l'acide glycyrrhétinique est un extrait de réglisse (*Glycyrrhiza spp*.). Dans un mode de réalisation préféré, la composition de l'invention comprend un extrait de *Glycyrrhiza*, de préférence choisi parmi *Glycyrrhiza uralensis*, *Glycyrrhiza inflata* et *Glycyrrhiza glabra.* Selon un mode de réalisation particulier, il s'agit d'un extrait de racines. A titre d'exemple, l'acide glycyrrhétinique purifié, de pureté au moins égale à 90% en poids, commercialisé par INDENA ou MARUZEN sous la dénomination INCI Glycyrrhetinic acid peut être mis en œuvre dans le cadre de l'invention.

De manière avantageuse, l'acide glycyrrhétinique, ou l'un de ses dérives ou sels, représente de à 0,01% à 1% en poids de la composition, avantageusement de 0,1% à 0,5% en poids de la composition, encore plus avantageusement 0,3% en poids de la composition.

Comme déjà dit, l'association de la DHM et d'un sel de zinc est privilégiée selon la présente demande.

Toutefois, il est possible d'ajouter à ces deux actifs d'autres ingrédients présentant une activité d'intérêt dans le cadre de la prise en charge des peaux ou des cuirs chevelus gras ou atteints d'acné, en particulier choisis parmi ceux listés ci-dessus. Ainsi et selon cet aspect préféré, la composition de la présente demande comprend de la dihydromyricétine, un sel de zinc, avantageusement le gluconate de zinc, et au moins un ingrédient choisi parmi :

- la biochanine A, ou un extrait végétal comprenant de la biochanine A, avantageusement un extrait de trèfle rouge;
- un extrait de *Ginkgo biloba,* avantageusement un extrait de *Ginkgo biloba* comprenant des flavonoïdes ;
- un méroterpène, avantageusement du bakuchiol ;
- un polyol, avantageusement choisi parmi le xylitol, le sorbitol et le mannitol ;
- un antioxydant lipophile, avantageusement choisi parmi le propyl gallate, l'octyl gallate et le dodécyl gallate ;
- un agent kératolytique, avantageusement choisi parmi l'acide salicylique, l'acide glycolique, l'acide citrique, l'acide malique et l'acide lactique ;
- l'acide glycyrrhétinique ou l'un de ses dérivés ou sels.

Avantageusement, ces différents ingrédients sont ajoutés, seuls ou en combinaison, dans les gammes de concentrations mentionnées ci-dessus en rapport avec chacun d'eux.

Selon un mode de réalisation privilégié, la composition de la présente demande comprend de la DHM, un sel de zinc, avantageusement du gluconate de zinc, et de la biochanine A ou un extrait végétal comprenant de la biochanine A, avantageusement un extrait de trèfle rouge.

Il est possible d'ajouter à ces trois actifs d'autres ingrédients présentant une activité d'intérêt parmi ceux déjà listés. Ainsi et selon cet aspect préféré, la composition de la présente demande comprend de la dihydromyricétine, un sel de zinc, avantageusement le gluconate de zinc, de la biochanine A ou un extrait végétal comprenant de la biochanine A, avantageusement un extrait de trèfle rouge, et au moins un ingrédient choisi parmi :

- un extrait de Ginkgo biloba, avantageusement un extrait de Ginkgo biloba comprenant des flavonoïdes ;
- un méroterpène, avantageusement du bakuchiol ;
- un polyol, avantageusement choisi parmi le xylitol, le sorbitol et le mannitol ;
- un antioxydant lipophile, avantageusement choisi parmi le propyl gallate, l'octyl gallate et le dodécyl gallate ;
- un agent kératolytique, avantageusement choisi parmi l'acide salicylique, l'acide glycolique, l'acide citrique, l'acide malique et l'acide lactique ;
- l'acide glycyrrhétinique ou l'un de ses dérivés ou sels.

Bien sûr, tout autre actif de grade cosmétique ou pharmaceutique, ayant une activité d'intérêt dans le cadre des applications visées par la présente demande, peut être incorporé dans la composition selon l'invention, tel que dérivé de vitamine, antioxydant hydrophile, anti-inflammatoire...

Selon l'invention, la composition est une composition cosmétique ou dermatologique, en d'autres termes est destinée à un usage cosmétique ou dermatologique. Par conséquent et avantageusement, l'ensemble des ingrédients présents dans une telle composition doivent être acceptables dans ces domaines d'application.

Au vu des applications visées, une composition selon l'invention est préférentiellement destinée à être appliquée de manière topique, avantageusement au niveau de la peau ou du cuir chevelu.

Ainsi et de manière privilégiée, la composition selon l'invention se présente sous une forme adaptée à l'administration par voie topique cutanée: crème, émulsion, avantageusement huile dans eau (H/E), eau dans huile (E/H) ou eau dans silicone (E/S), solution, suspension, gel, lait ou lotion, eau micellaire, de préférence sous forme de crème, gel,

lotion ou émulsion, avantageusement huile dans eau (H/E).

Par conséquent, la présente composition peut contenir tout additif ou excipient adapté à sa formulation et à son application, tel que par exemple agents de mise en suspension, émulsifiants, polymères anioniques, cationiques ou non-ioniques, amphotères, protéines, vitamines, tensioactifs, huiles minérales ou végétales, antioxydants, cires, gommes, résines, agents épaississants, acidifiants ou alcalinisants, stabilisateurs de pH, agents anti-UV, filtres et écrans solaires, conservateurs, parfums, colorants, adjuvants classiques de la cosmétique et de la dermatologie.

Comme déjà dit, la composition selon l'invention est particulièrement adaptée au traitement des pathologies cutanées associées à la présence de *P. acnes.* Plus généralement, elle présente un intérêt dans la prise en charge de sujets présentant au moins un des symptômes de l'acné, quel que soit son stade de développement, ou susceptibles de le développer, en particulier chez les sujets à peaux grasses et/ou à tendance acnéique. La composition selon l'invention est aussi d'intérêt pour la prévention ou la prise en charge des séquelles cutanées dues à l'acné, en particulier la formation des cicatrices atrophiques de l'acné.

Selon un autre aspect, l'invention vise donc une composition comprenant de la dihydromyricétine et un sel de zinc pour son utilisation dans la prévention et/ou le traitement de l'acné. L'invention vise en outre une composition telle que décrite ci-dessus pour son utilisation dans la prévention et/ou le traitement des cicatrices atrophiques de l'acné.

La composition de l'invention peut aussi être utilisée dans des applications non thérapeutiques, telles que des applications cosmétiques, en particulier celles visant les peaux grasses ou les cuirs chevelus à tendance acnéique. Ainsi et selon un autre aspect, l'invention concerne un procédé de traitement cosmétique des peaux grasses ou à tendance acnéique et/ou du cuir chevelu à tendance acnéique consistant à appliquer sur la peau et/ou sur le cuir chevelu une composition selon l'invention.

Dans le cadre de la présente demande et comme il sera illustré ci-après, des propriétés intéressantes et inédites de la DHM combinée à un sel de zinc tel que le gluconate de zinc, et de manière particulièrement avantageuse combinée à la fois à un sel de zinc tel que le gluconate de zinc et à la biochanine A, ont été mises en évidence. Selon un autre aspect est également visée l'utilisation d'une telle composition comme :

- agent séborégulateur, notamment via son action inhibitrice sur l'hyperproduction de sébum par les sébocytes ; et/ou
- agent fluidifiant du sébum, notamment via son activité protectrice du squalène du sébum contre l'oxydation ; et/ou
- agent apte à prévenir la formation de cicatrices atrophiques de l'acné, notamment via son action inhibitrice sur l'activité des enzymes de type MMP
- agent antimicrobien, notamment antibactérien vis-à-vis de *P. acnes.*

La manière dont l'invention peut être réalisée et les avantages qui en découlent ressortiront mieux des exemples de réalisation qui suivent, donnés à titre indicatif et non limitatif, à l'appui des figures annexées.

<u>La figure 1</u> représente l'effet de la dihydromyricétine (DHM) à 3 concentrations (0,0005%, 0,001% et 0,003% en poids) sur la synthèse des lipides induite par l'IGF-1 (20 ng/ml) dans des sébocytes après 14 jours de culture.

<u>La figure 2</u> représente l'effet de la DHM à 0,001% en poids et/ou du gluconate de zinc à 0,001% en poids sur la synthèse des lipides induite par l'IGF-1 dans des sébocytes après 14 jours de culture.

<u>La figure 3</u> représente l'effet de la DHM (0,002% et 0,005% en poids), du gluconate de zinc (0,1%) et de leur association (0,002% et 0,1% en poids, respectivement) sur l'activité collagénase d'une MMP (« Matrix Metallo-Proteinase ») issue de *Clostridium histolyticum.*

## Exemples de réalisation

### I/ Exemples de formulation

Les quantités indiquées sont données en pourcentage en poids.

#### Exemple 1 : émulsion eau dans silicone (E/S)

| *Nom INCI* | *% INCI* |
|---|---|
| Glycerin | 10,00 |

(suite)

| *Nom INCI* | *% INCI* |
|---|---|
| Cyclopentasiloxane | 10,00 |
| Cyclohexasiloxane | 5,00 |
| Glycolic acid | 4,00 |
| Dipropylene glycol | 5,00 |
| Zinc gluconate | 3,00 |
| Polymethylsilsesquioxane | 2,50 |
| Dimethicone | 2,50 |
| Peg-10 dimethicone | 2,00 |
| Propylene glycol | 1,60 |
| Dimethicone/polyglycerin-3 crosspolymer | 0,80 |
| Sodium hydroxide | 0,70 |
| Glycyrrhetinic acid | 0,30 |
| Bakuchiol | 0,25 |
| Ginkgo biloba leaf extract | 0,10 |
| Sodium metabisulfite | 0,10 |
| Xylitol | 0,10 |
| Trifolium pratense (clover) leaf extract | 0,06 |
| Xanthan gum | 0,05 |
| Dihydromyricetin | 0,01 |
| Aqua/eau | qsp. 100 |

**Exemple 2 : émulsion huile dans eau (H/E)**

| *Nom INCI* | *% INCI* |
|---|---|
| Glycerin | 10,000000 |
| Coco-caprylate/caprate | 7,496625 |
| Cyclopentasiloxane | 5,062500 |
| Dipropylene glycol | 5,000000 |
| Zinc gluconate | 3,000000 |
| Glycolic acid | 2,030000 |
| Citric acid | 2,000000 |
| Tribehenin peg-20 esters | 2,000000 |
| Propylene glycol | 1,552000 |
| Sodium hydroxide | 1,300000 |
| Glyceryl stearate | 1,000000 |
| Silica | 1,000000 |
| Polyacrylate crosspolymer-6 | 0,700000 |

(suite)

| Nom INCI | % INCI |
|---|---|
| Sodium polyacrylate | 0,500000 |
| Glycyrrhetinic acid | 0,300000 |
| Bakuchiol | 0,250000 |
| Fragrance (parfum) | 0,250000 |
| Sodium metabisulfite | 0,100000 |
| Xylitol | 0,100000 |
| Trifolium pratense (clover) leaf extract | 0,060000 |
| Dihydromyricetin | 0,010000 |
| Ginkgo biloba leaf extract | 0,008000 |
| Aqua/eau | Qsp. 100 |

**III Protection de l'oxydation du squalène par la dihydromyricétine (DHM)**

II. 1 INTRODUCTION

L'objectif de ce test est d'évaluer l'inhibition par la DHM de l'oxydation du squalène, induite par le peroxyde d'hydrogène ($H_2O_2$). Le Trolox (6-Hydroxy-2,5,7,8-tétraméthylchroman-2-carboxylic acid), un équivalent hydrosoluble de la vitamine E, est un antioxydant connu et est utilisé dans ces tests en tant que témoin positif.

II.2 MATERIELS ET METHODES

Le squalène (Sigma Aldrich) est dans un premier temps solubilisé dans une chambre réactionnelle.
L'oxydation du squalène est induite par l'ajout d'une solution de peroxyde d'hydrogène ($H_2O_2$ à une concentration finale de 6%).
Les actifs devant être évalués sont ajoutés au mélange réactionnel. Ainsi, le Trolox à 0,8% en poids est utilisé comme témoin positif de la protection contre l'oxydation du squalène.
Un contrôle positif, correspondant au squalène incubé en présence d'$H_2O_2$, et un contrôle négatif, correspondant au squalène non exposé à l'$H_2O_2$, sont également réalisés.
Le mélange obtenu est mis sous agitation pendant une heure, à température ambiante et à l'abri de la lumière.
Le squalène est ensuite extrait du milieu réactionnel à l'aide d'une extraction liquide/liquide. Après évaporation de la solution organique, le résidu est solubilisé dans une solution de chloroforme et le squalène est analysé par une méthode de chromatographie GC/MS.
La mesure de la concentration en squalène permet d'estimer la quantité de squalène ayant été oxydée au cours de la réaction. En effet et selon la technique de mesure utilisée, seule la forme non oxydée est détectée.

II.3 RESULTATS ET CONCLUSIONS

Les résultats obtenus sont présentés dans le tableau 1 ci-dessous :

**Tableau 1** : Effet du trolox (0,8%) et de la DHM (0,005%, 0,01% et 0,05%) sur la protection contre l'oxydation du squalène induite par le peroxyde d'hydrogène.

| Essai | Concentration (% en poids) | Echantillon | Squalène (µg/ml) | Squalène Moyenne | Squalène CV (%) | Indice de protection (%) |
|---|---|---|---|---|---|---|
| 1 | / | Contrôle Positif 1 | 10,17 | 9,79 | 3,62 | 13,20 |
| 2 | | | 9,48 | | | |
| 3 | | | 9,71 | | | |

(suite)

| Essai | Concentration (% en poids) | Echantillon | Squalène (μg/ml) | Squalène Moyenne | Squalène CV (%) | Indice de protection (%) |
|---|---|---|---|---|---|---|
| 1 | / | Contrôle Positif 2 | 10,04 | 9,92 | 5,20 | 13,38 |
| 2 | | | 9,36 | | | |
| 3 | | | 10,37 | | | |
| 1 | / | Contrôle Négatif 1 | 73,58 | 74,87 | 1,53 | / |
| 2 | | | 75,75 | | | |
| 3 | | | 75,29 | | | |
| 1 | / | Contrôle Négatif 2 | 72,23 | 73,45 | 1,67 | / |
| 2 | | | 74,68 | | | |
| 3 | | | 73,42 | | | |
| 2 | | | 40,09 | | | |
| 3 | | | 39,12 | | | |
| 1 | 0,8 | Trolox | 31,41 | 31,85 | 1,31 | 42,94 |
| 2 | | | 31,88 | | | |
| 3 | | | 32,25 | | | |
| 2 | | | 34,60 | | | |
| 3 | | | 32,49 | | | |
| 1 | 0,005 | DHM | 19,84 | 21,24 | 6,32 | 28,64 |
| 2 | | | 21,37 | | | |
| 3 | | | 22,51 | | | |
| 1 | 0,01 | | 27,01 | 26,10 | 4,26 | 35,19 |
| 2 | | | 24,86 | | | |
| 3 | | | 26,42 | | | |
| 1 | 0,05 | | 34,44 | 33,58 | 2,37 | 45,28 |
| 2 | | | 32,88 | | | |
| 3 | | | 33,41 | | | |
| CV : coefficient de variation | | | | | | |

On constate que la DHM possède une activité protectrice contre l'oxydation du squalène induite par le peroxyde d'hydrogène, qui est par ailleurs dose-dépendante. A une concentration de 0,05%, la DHM possède une activité protectrice contre l'oxydation du squalène comparable à celle du Trolox, lorsque celui-ci est utilisé à une concentration 16 fois plus élevée (0,8%).

Ces expériences démontrent un effet protecteur de la DHM vis-à-vis de l'oxydation du squalène, comparable à celui observé en présence d'antioxydants connus.

## III/ Effet de la DHM et/ou du gluconate de zinc sur la production de lipides dans un modèle de sébocytes humains

III.1 INTRODUCTION

Ce test est basé sur l'utilisation de la lignée de sébocytes SEB0662 décrite par Barrault *et al.*., 2012.

La présente étude vise à étudier l'effet de la DHM et/ou du gluconate de zinc (Zn) sur l'inhibition de la synthèse des lipides, induite par l'ajout d'IGF-1, dans les sébocytes. Cet effet est suivi par marquage, à l'aide d'une sonde lipidique

fluorescente couplée au Bodipy®, des sébocytes SEB0662 cultivés en monocouche pendant 14 jours en milieu de différenciation (supplémenté en IGF-1 à 20 ng/ml).

III.2. MATERIELS ET METHODES

III.2.1. Modèles biologiques

Dans cette étude, on utilise la lignée de sébocytes humains SEB0662.

Les cellules sont cultivées dans le milieu de culture classique des sébocytes, à 37°C et en présence de 5% de $CO_2$. Les cellules sont utilisées au $7^{ème}$ passage.

Pour l'évaluation des actifs, un milieu dit de différenciation (milieu de culture classique supplémenté en dihydrotestostérone, albumine bovine sérique, acide linoléïque, glucose, sérum de veau fœtal, hydrocortisone et agonistes des PPAR (« Peroxysome Proliferator Activated Receptor »)), complémenté en IGF-1 à 20 ng/ml, est utilisé. L'ajout d'IGF-1 stimule la production de lipides par les sébocytes.

III.2.2. Cultures et traitements

Les sébocytes SEB0662 sont ensemencés à très forte densité (50000 cellules/puits) en plaque 96 puits et cultivés en milieu de culture classique pendant 24 heures. Après incubation, le milieu est remplacé par le milieu de différenciation décrit plus haut, contenant ou non les composés à tester. Les contrôles suivants sont réalisés: milieu de culture classique et milieu de différenciation dépourvu d'IGF-1.

La sélection des doses d'actifs est réalisée grâce à un test de viabilité cellulaire au MTT. On retient les doses qui permettent de maintenir une viabilité supérieure à 90%.

Après 7 jours de culture, les milieux contenant de l'IGF-1 sont à nouveau supplémentés en IGF-1 (20 ng/ml) et en composé(s) à tester.

A l'issue d'un total de 14 jours de culture, le marquage des gouttelettes lipidiques est réalisé comme décrit ci-dessous.

Toutes les conditions expérimentales sont répétées trois fois.

III.2.3. Marquage *in situ* des gouttelettes lipidiques

Le milieu de différenciation est éliminé et les cellules sont rincées, fixées et perméabilisées. Les gouttelettes lipidiques contenues dans les cellules sont ensuite marquées à l'aide d'une sonde lipidique fluorescente couplée au Bodipy®. En parallèle, les noyaux des cellules sont colorés par le Hoechst 33258 (bis-benzimide).

L'acquisition des images est réalisée avec un système d'imagerie à haute résolution, INCell Analyzer™ I000 (GE Healthcare). Pour chaque puits, 10 saisies d'images numérisées sont effectuées pour chaque marquage.

Les marquages sont quantifiés par mesure de l'intensité de fluorescence de la sonde rapportée au nombre de noyaux identifiés par le Hoechst (intégration des données numériques par le logiciel Developer Toolbox 1 .5, GE Healthcare).

III.2.4 Traitement des données

Les données brutes sont transférées et traitées sous le logiciel Microsoft Excel®.

Les comparaisons intergroupes sont réalisées à l'aide du test T de Student bilatéral non apparié. Les analyses statistiques sont fournies à titre indicatif.

<u>Formules utilisées pour l'analyse des résultats</u> :

L'erreur standard de la moyenne (esm) représente l'écart de ln moyenne de l'échantillon par rapport à la moyenne de la population. L'esm est calculée en divisant l'écart-type (Sd) par la racine carrée de la taille de l'échantillon (sqrt(n)).

$$\text{Erreur standard de la moyenne (esm)} = \text{écart-type (Sd)/sqrt(n)}$$

$$\text{Pourcentage de viabilité :} \quad \text{viabilité (\%)} = (DO_{\text{composé}} / DO_{\text{témoin}}) \times 100$$

$$\text{Pourcentage de stimulation : Stimulation (\%)} = [\ (\text{Valeur/moyenne du témoin}) \times 100\ ] - 100$$

$$\text{Pourcentage d'inhibition : Inhibition (\%)} = 100 - [\ (\text{Valeur/moyenne du témoin}) \times 100\ ]$$

III.3 RESULTATS ET CONCLUSIONS

III.3.1 Test de cytotoxicité

Dans les conditions de culture utilisées, la DHM seule, testée jusqu'à la dose de 0,003%, ne montre pas d'effet cytotoxique. Les doses de 0,0005%, 0,001% et 0,003% sont sélectionnées pour la réalisation des tests.
Le gluconate de zinc ne présente pas de cytotoxicité à la dose de 0,001%. La dose de 0,001% est sélectionnée pour la réalisation des tests.
En association, la présence de gluconate de zinc à 0,001% et de DHM à 0,001% permet de maintenir une viabilité cellulaire toujours supérieure à 90%.

III.3.2 Effets sur la lipogenèse

Le milieu de différenciation supplémenté en IGF-1 induit, dans les conditions du test, une augmentation du nombre de vésicules lipidiques (168% du témoin). Ce résultat attendu valide les conditions expérimentales.
Toutes les conditions testées pour la DHM (3 concentrations) induisent une diminution significative de l'intensité du marquage Bodipy® et une diminution de la formation des gouttelettes lipidiques. Ainsi et comme illustré à la figure 1, la DHM inhibe la synthèse des lipides induite par l'IGF-1 :

- de 19% à la dose de 0,0005% ;
- de 12% à la dose de 0,001% ; et
- de 17% à la dose de 0,003%.

Par ailleurs et comme montré à la figure 2, le gluconate de zinc à 0,001% inhibe également de 15% environ la synthèse de lipides. De manière remarquable, l'ajout de DHM à 0,001% permet encore d'améliorer l'inhibition de la synthèse des lipides induite par IGF-1.

**IV/ Effet de la DHM et/ou du gluconate de zinc sur l'activité collagénase de la MMP-1 par un test *in tubo***

IV.1 INTRODUCTION

Les tests effectués permettent d'évaluer l'effet de la DHM, du gluconate de zinc, et de leur association sur l'activité collagénase d'une MMP.

IV.2. MATERIELS ET METHODES

IV.2.1 Principe du test

L'activité anti-collagénase est mesurée grâce au kit MMP-1 (Molecular Probes ; Réf E12055). Le principe est basé sur la capacité d'une enzyme de la famille des MMP (« Marix Metallo-Proteinase ») zinc-dépendantes, en l'occurrence la collagénase issue de *Clostridium histolyticum,* à hydrolyser son substrat (collagène-I) pour donner des composés fluorescents mesurables.
En présence d'un inhibiteur, l'action de l'enzyme est altérée empêchant l'hydrolyse de son substrat. La 1,10 phénanthroline est utilisée comme le témoin positif d'inhibition de l'enzyme. La fluorescence émise à 535 nm est mesurée après excitation à 485 nm.

IV.2.2 Dilutions des actifs et contrôles

La 1,10-phenanthroline monohydrate (10mM), un inhibiteur connu des MMP fourni dans le kit, est utilisée comme contrôle positif.
Les contrôles négatifs suivants ont été réalisés :

- Témoin $H_2O$ : ce témoin a été utilisé pour solubiliser la 1-10-phenantroline monohydrate ; il représente arbitrairement 100% d'activité de l'enzyme par rapport aux résultats obtenus avec ce contrôle.
- Témoin solvant (DMSO 1%) : quantité de solvant inclus dans l'ensemble des actifs et associations à tester ; il représente arbitrairement 100% d'activité de l'enzyme par rapport aux résultats obtenus avec les actifs et associations testés.

Le gluconate de zinc a été testé à la concentration de 0,1% (en poids).

La DHM a été testée aux concentrations suivantes : 0,002% et 0,05% (en poids). L'association gluconate de zinc 0,1% + DHM 0,002% a également été testée.

IV.2.3. Analyses statistiques

Les analyses quantitatives sont exprimées sous forme de moyenne ± l'écart type. La significativité statistique est déterminée par un test de Student. Les différences sont considérées comme statistiquement significatives entre l'actif testé et son témoin solvant à partir de $p<0.05$. (NS : $p<0.05$ ; * : $p<0.01$ ; ** : $p<0.001$ ***).

IV.3. RESULTATS ET CONCLUSIONS

Les activités anti-collagénase des contrôles, des actifs et de leur association sont évaluées par un test de mesure de l'activité MMP-1. Les résultats sont présentés à la Figure 3.

L'analyse des résultats révèle les effets suivants :

- Comme attendu, le témoin positif d'inhibition de la collagénase (1-10-phénanthroline monohydrate 10mM) inhibe significativement l'activité collagénase (inhibition observée: 79,6%).
- Le gluconate de zinc testé à 0,1% inhibe significativement l'activité collagénase de la MMP-1 (inhibition observée: 80,2 %).
- La DHM, testée à 0,002% et 0,05%, inhibe significativement et de manière dose-dépendante l'activité collagénase de la MMP (inhibition observée: 3,5% et 30,1%, respectivement).
- L'association gluconate de zinc 0,1% + DHM 0,002% inhibe très significativement l'activité collagénase de la MMP (inhibition observée de 90,7%), révélant une synergie entre les 2 composés dans ces conditions.

**V/ Effet de la DHM, du gluconate de zinc, et de l'extrait de trèfle rouge titré en biochanine A sur la croissance de *P. acnes***

V.1 - INTRODUCTION

L'objet de cette étude est d'évaluer l'activité inhibitrice de composés vis-à-vis de la souche *Propionibacterium acnes* selon les principes de la méthode décrite par Lens-Lisbonne *et al.* (1987).

La méthode de Lens-Lisbonne *et al.* permet de tester en milieu liquide le pouvoir antibactérien de substances faiblement solubles et/ou très volatiles, et permet d'éviter les méthodes par diffusion ou en phase vapeur. Brièvement, elle consiste à mettre en présence des volumes égaux de produit à tester et de milieu inoculé, à différentes concentrations. Pour ce faire, les produits à tester et les milieux inoculés sont préalablement préparés à une concentration deux fois plus importante (« concentration double ») que la concentration finale désirée. L'activité inhibitrice est évaluée à partir des mesures d'absorbance du mélange, comparées aux conditions contrôle. Cette méthode peut être mise en œuvre dans des volumes réactionnels importants, par exemple d'environ 20 ml, auquel cas elle est désignée « macrométhode ». Alternativement, elle peut être mise en œuvre en utilisant de très faibles volumes réactionnels, d'environ 200 µL, auquel cas elle est désignée « microméthode ». La microméthode, qui permet l'utilisation de microplaques et de matériel automatisé, a été utilisée dans cet exemple.

V.2 MATERIELS ET METHODES

V.2.1 - Identification des échantillons

**Tableau 2** : Identification des échantillons

| Actif testé | Composition | Lot |
|---|---|---|
| Zinc | Gluconate de zinc 100% | 152913 |
| Extrait - biochanine A | 49% propylène glycol<br>49% eau<br>2% extrait hydroglycolique de trèfle rouge comprenant de la biochanine A | TRP140701 |
| DHM | Dihydromyricétine 100% | 1505216 |
| DMSO (témoin) | diméthylsulfoxyde | 14C2240015 |

V.2.2- Principe de l'essai

Un bouillon nutritif est préparé, qui correspond à du milieu liquide dans lequel sont ensemencées les bactéries d'intérêt. Parallèlement, des dilutions successives des produits à tester sont préparées.

Les produits suivants sont mis en contact dans un puit de microplaque adaptée:

- 100 μl du produit à tester à concentration double de la concentration finale, et
- 100 μl de bouillon nutritif à une concentration double titrant environ entre 2 et $6.10^5$ UFC/ml.

Après incubation de la microplaque pendant une durée définie, la densité optique à 620 nm est mesurée. Les résultats sont exprimés en pourcentage de croissance calculé par rapport à un témoin de croissance et un témoin d'absorbance selon l'équation suivante:

*Pourcentage de croissance = (DO mesurée du produit à la concentration C - DO mesurée du témoin d'absorbance produit à la concentration C)/DO mesurée témoin de croissance*

Le témoin de croissance correspond à un mélange de 100 μL d'agar 1‰ et de 100 μl de bouillon nutritif à la même concentration double que celle utilisée dans le mélange réactionnel comprenant le produit à tester, incubé dans les mêmes conditions que le mélange réactionnel comprenant le produit à tester.

Le témoin d'absorbance correspond à un mélange de 100 μL du produit à tester à la même concentration double que celle testée, et de 100 μl de bouillon nutritif stérile à la même concentration double que celle utilisée dans le mélange réactionnel comprenant le produit à tester, incubé dans les mêmes conditions que le mélange réactionnel comprenant le produit à tester.

Est considérée comme inhibitrice la première concentration en produit testé permettant l'obtention d'un pourcentage de croissance inférieur ou égal à 20 %.

V.2.3- Conditions expérimentales

Préparation du bouillon nutritif :

Souche microbienne : *Propionibacterium acnes* ATCC 69.19.
La souche provient de la collection de la Collection de l'Institut Pasteur et est entretenue au laboratoire selon les exigences de la norme NF EN 12353.
Composition du milieu liquide: Bouillon Brain Heart Infusion (BHI) à double concentration.

Préparation des dilutions des composés à tester :

Les solutions mères sont préparées en agar 1 ‰ ou en DMSO. Des dilutions successives en agar 1‰ sont effectuées.

Le test est effectué sur microplaque 96 puits. En synthèse, 30 puits sont utilisés pour les témoins d'absorbance ; 30 puits sont utilisés pour les essais. 3 puits sont réalisés pour chaque concentration et, en outre, les essais et témoins sont effectués en duplicata.

Une microplaque témoin de croissance est réalisée séparément.

Température d'incubation de la microplaque : 36° C.
Durée d'incubation de la microplaque: 3 à 5 jours en anaérobiose.

V.2.4 - Expression des résultats

Pour chaque concentration en matière première testée et chaque test, la moyenne des valeurs de densité optique mesurée sur les 3 puits est effectuée et les pourcentages de croissance sont calculés.
Ces valeurs sont reportées sur un graphique (pourcentage de croissance en fonction de la concentration) afin d'étudier les profils d'activité de chaque matière première testée.
On déduit de ces valeurs la CMI de chacun des actifs testés, telle que définie plus haut, c'est-à-dire la première concentration en produit testé permettant l'obtention d'un pourcentage de croissance inférieur ou égal à 20 %.

V.3- RESULTATS

Les résultats sont présentés dans le tableau suivant :

**Tableau 3** : résultats

| Actif testé | Solvant | CMI microméthode (%-m/v) | |
|---|---|---|---|
| | | Test 1 | Test 2 |
| Zinc | Agar | 0,05 | 0,05 |
| Extrait - biochanine A | Agar | Non déterminée | Non déterminée |
| DHM | DMSO | ≈0,025 | 0,025 |
| DMSO (témoin) | - | >5 | >5 |

V.4 CONCLUSION

Comme attendu, aucune CMI ne peut être déterminée pour le DMSO (solvant) à des concentrations inferieures à 5% (concentration maximale de DMSO utilisée pour solubiliser la DHM qui ne peut pas être solubilisé dans l'agar).
Aucune CMI ne peut être déterminée pour l'actif «Extrait-biochanine A» aux concentrations testées (jusqu'à 3%).
De façon surprenante, la CMI de l'actif «DHM», une molécule qui n'est pas connue pour avoir un effet antibactérien, est significative et inférieure à celle du gluconate de zinc («zinc»), un agent antimicrobien traditionnellement utilisé en dermocosmétique, en particulier pour ses propriétés d'inhibition de la croissance de *P. acnes.*

## REFERENCES

BARRAULT C, DICHAMP I, GARNIER J, PEDRETTI N, JUCHAUX F, DEGUERCY A, AGIUS G, BERNARD FX. Immortalized sebocytes can spontaneously differentiate into a sebaceous-like phenotype when cultured as a 3D epithelium. Exp Dermatol. 2012 Apr;21(4):314-6.

CAPPEL M, MAUGER D, THIBOUTOT D. Correlation between serum levels of insulin-like growth factor 1, dehydroepiandrosterone sulfate, and dihydrotestosterone and acne lesion counts in adult women. Arch Dermatol. Mars 2005;141(3):333-8.

CHIBA K, SONE T, KAWAKAMI K, ONOUE M. Skin roughness and wrinkle formation induced by repeated application of squalene-monohydroperoxide to the hairless mouse. Exp Dermatol., Décembre 1999, vol. 8 (6), 471-9.

CHIBA K, YOSHIZAWA K, MAKINO I, KAWAKAMI K, ONOUE M. Comedogenicity of squalene monohydroperoxide in the skin after topical application. J Toxicol Sci 2000; 25: 77-83.

CHIVOT M, PAWIN H, BEYLOT C, CHOSIDOW O, DRENO B, FAURE M, POLI F, REVUZ J, cicatrices d'acné : épidémiologie, physiopathologie, clinique, traitement. Acne scars: epidemiology, physiopathology, clinical features and treatment Ann Dermatol Venereol Oct. 2006: Vol 133, 813-824.

DEPLEWSKI D, ROSENFIELD RL. Growth hormone and insulin-like growth factors have different effects on sebaceous cell growth and differentiation. Endocrinology. 1999 Sep;140(9):4089-94.

DEPLEWSKI D, ROSENFIELD RL. Role of hormones in pilosebaceous unit development. Endocr Rev. 2000 Aug;21(4):363-92. Review.

GERATS AG, DE VLAMING P, DOODEMAN M, AL B, SCHRAM AW. Genetic control of the conversion of dihydroflavonols into flavonols and anthocyanins in flowers of Petunia hybrida. Planta. 1982 Aug;155(4):364-8. doi: 10.1007/BF00429466.

HALL MJ, MIDDLETON, RF, WESTMACOTT D The fractional inhibitory concentration (FIC) index as a measure of synergy J. Antimicrob. Chemother. (1983) 11 (5): 427-433 doi:10.1093/jac/11.5.427.

HORTON R, PASUPULETTI V, ANTONIPILLAI I. Androgen induction of steroid 5 alpha-reductase may be mediated via insulin-like growth factor-I. Endocrinology. 1993 Aug;133(2):447-51.

ISARD O, KNOL AC, ARIES MF, NGUYEN JM, KHAMMARI A, CASTEX-RIZZI N, DRENO B. Propionibacterium acnes activates the IGF-1/IGF-1R system in the epidermis and induces keratinocyte proliferation. J Invest Dermatol. 2011 Jan;131(1):59-66. doi: 10.1038/jid.2010.281. Epub 2010 Oct 7.

LE LAIN R, NICHOLLS PJ, SMITH HJ, MAHARLOUIE FH. Inhibitors of human and rat testes microsomal 17beta-hydroxysteroid dehydrogenase (17beta-HSD) as potential agents for prostatic cancer. J Enzyme Inhib. 2001 Jan;16(1):35-45.

LENS-LISBONNE C, . CREMIEUX C, MAILLARD C, BALANSARD G Evaluation activité antibactérienne des huiles essentielles J. Pharm. Belg., 1987, 42, 5, 297-302.

OHNO S., MATSUMOTO N, WATANABE M, NAKAJIN S. Flavonoid inhibition of overexpressed human 3beta-hydroxysteroid dehydrogenase type II. J Steroid Biochem Mol Biol. 2004 Feb;88(2):175-82.

ORFANOS CE, ZOUBOULIS CC. Oral retinoids in the treatment of seborrhoea and acne. Dermatology. 1998;196(1):140-7.

OTTAVIANI M, CAMERA E, PICARDO M. Lipidmédiators in acne. Mediators Inflamm, 25 Août 2010.

OTTAVIANI M, ALESTAS T, FLORI E, MASTROFRANCESCO A, ;ZOUBOULIS CC, PICARDO, M. Peroxidated squalene induces the production of inflammatory mediators in HaCaT keratinocytes: a possible role in acne vulgaris. J Invest Dermatol., 15 Juin 2006, vol. 126 (11), 2430-7.

PICARDO M, OTTAVIANI M; CAMERA E; MASTROFRANCESCO A. Sebaceous gland lipids. Dermatoendocrinol, Mars 2009, vol. 1 (2), 68-71.

SAINT-LÉGER D et al. A possible role for squalene in the pathogenesis of acne. In vivo study of squalene oxides in skin surface and intra-comedonal lipids of acne patients. Br J Dermatol, 1986, vol. 114, 543-552.

SHEN Y, LINDEMEYER AK, GONZALEZ C, SHAO XM, SPIGELMAN I, OLSEN RW, LIANG J. Dihydromyricetin as a novel anti-alcohol intoxication medication. J Neurosci. 2012 Jan 4;32(1):390-401.

STAMATIADIS D, BULTEAU-PORTOIS MC, MOWSZOWICZ I. 1988 Inhibition of 5-$\alpha$-réductase activity in human skin by Zinc and azelaic acid. British Journal of Dermatology 119(5): 627-632.

THIELE JJ, WEBER SU, PACKER L. Sebaceous gland secretion is a major physiologie route of vitam in E delivery to skin. J Invest Dermatol., Déc. 1999, vol. 113 (6), 1006-10.

WU S, LIU B, ZHANG Q, LIU J, ZHOU W, WANG C, LI M, BAO S, ZHU R Dihydromyricetin reduced Bcl-2 expression via p53 in human hepatoma HepG2 cells. PLoS One. 2013 Nov 4;8(11):e76886.

## Revendications

1. Composition cosmétique ou dermatologique comprenant de la dihydromyricétine, un sel de zinc et un antioxydant lipophile, **caractérisée en ce que** l'antioxydant lipophile est choisi parmi le propyl gallate, l'octyl gallate et le dodécyl gallate.

2. Composition selon la revendication 1, **caractérisée en ce qu'**elle comprend en outre de la biochanine A ou un extrait végétal comprenant de la biochanine A, avantageusement un extrait de trèfle rouge.

3. Composition selon l'une des revendications précédentes, **caractérisée en ce que** la dihydromyricétine représente de 0,0001% à 10% en poids de la composition, avantageusement de 0,001% à 2%.

4. Composition selon l'une des revendications précédentes, **caractérisée en ce que** le sel de zinc est le gluconate de zinc.

5. Composition selon l'une des revendications précédentes, **caractérisée en ce que** le sel de zinc représente de 0,1 à 10% en poids de la composition, avantageusement de 1 à 5%.

6. Composition selon l'une des revendications précédentes, **caractérisée en ce qu'**elle comprend en outre au moins un ingrédient choisi parmi :

   - un extrait de *Ginkgo biloba,* avantageusement un extrait de *Ginkgo biloba* comprenant des flavonoïdes ;
   - un méroterpène, avantageusement du bakuchiol ;
   - un polyol, avantageusement choisi parmi le xylitol, le sorbitol et le mannitol ;
   - un agent kératolytique, avantageusement choisi parmi l'acide salicylique, l'acide glycolique, l'acide citrique, l'acide malique et l'acide lactique ;
   - l'acide glycyrrhétinique ou l'un de ses dérivés ou sels.

7. Composition selon l'une des revendications précédentes, **caractérisée en ce qu'**elle se présente sous la forme d'une crème, d'un gel, d'une lotion, ou d'une émulsion, avantageusement huile dans eau (H/E).

8. Composition cosmétique ou dermatologique comprenant de la dihydromyricétine et un sel de zinc pour son utilisation dans la prévention ou le traitement de l'acné.

9. Composition cosmétique ou dermatologique comprenant de la dihydromyricétine et un sel de zinc pour son utilisation comme :

   - agent séborégulateur ; et/ou
   - agent fluidifiant du sébum ; et/ou
   - agent apte à prévenir la formation des cicatrices atrophiques de l'acné ; et/ou
   - agent antimicrobien, notamment antibactérien vis-à-vis de *P. acnes.*

10. Composition cosmétique ou dermatologique pour son utilisation selon l'une des revendications 8 ou 9, telle que définie dans les revendications 1 à 7.

## Patentansprüche

1. Kosmetische oder dermatologische Zusammensetzung, die Dihydromyricetin, ein Zinksalz und ein lipophiles Antioxydans enthält, **dadurch gekennzeichnet, dass** das lipophile Antioxydans ausgewählt wird aus Propylgallat, Octylgallat und Dodecylgallat.

2. Zusammensetzung nach Anspruch 1, **dadurch gekennzeichnet, dass** sie außerdem Biochanin A enthält oder einen pflanzlichen Extrakt, der Biochanin A enthält, vorteilhafterweiese ein Rotklee- Extrakt.

3. Zusammensetzung nach irgendeinem der vorangehenden Ansprüche, **dadurch gekennzeichnet, dass** das Dihydromyricetin 0,0001 bis 10 Gewichts-% der Zusammensetzung bildet, vorteilhafterweise zwischen 0,001% und 2%.

4. Zusammensetzung nach irgendeinem der vorangehenden Ansprüche, **dadurch gekennzeichnet, dass** es sich bei dem Zinksalz um Zinkgluconat handelt.

5. Zusammensetzung nach irgendeinem der vorangehenden Ansprüche, **dadurch gekennzeichnet, dass** das Zinksalz 0,1 bis 10 Gewichts-% der Zusammensetzung, bildet, vorteilhafterweise zwischen 1 und 5%.

6. Zusammensetzung nach irgendeinem der vorangehenden Ansprüche, **dadurch gekennzeichnet, dass** sie außerdem einen Bestandteil enthält, bei dem es sich handeln kann um:

   - einen Extrakt aus *Ginkgo biloba,* vorteilhafterweise einen *Ginkgo biloba* - Extrakt mit Flavonoiden;
   - Ein Meroterpen, vorteilhafterweise Bakuchiol;
   - Ein Polyol, vorteilhafterweise ausgewählt aus Xylitol, Sorbitol und Mannitol;
   - Ein Keratolytikum, vorteilhafterweise ausgewählt aus Salicylsäure, Glycolsäure, Zitronensäure, Apfelsäure und Milschsäure;
   - Glycyrrhetinsäure oder eines ihrer Derivate oder Salze.

7. Zusammensetzung nach irgendeinem der vorangehenden Ansprüche, **dadurch gekennzeichnet, dass** sie die Form einer Creme, eines Gels, einer Lotion oder einer Emulsion, vorteilhafterweise Öl in Wasser (O/W), hat.

8. Kosmetische oder dermatologische Zusammensetzung, die Dihydromyricetin und ein Zinksalz enthält, zur Verwendung bei der Prävention oder Behandlung von Akne.

9. Kosmetische oder dermatologische Zusammensetzung, die Dihydromyricetin und ein Zinksalz enthält, zur Verwendung als:

   - sebumregulierender Wirkstoff; und/ oder
   - sebumverflüssigender Wirkstoff; und/ oder
   - Wirkstoff zur Verhinderung der Bildung atrophischer Aknenarben; und/ oder
   - antimikrobieller Wirkstoff, insbesondere antimikrobiell gegenüber *P. acnes.*

10. Kosmetische oder dermatologische Zusammensetzung zur Verwendung nach einem der Ansprüche 8 oder 9, wie definiert in den Ansprüchen 1 bis 7.

## Claims

1. A cosmetic or dermatological composition comprising dihydromyricetin, a zinc salt and a lipophilic antioxidant, **characterized in that** the lipophilic antioxidant is chosen from propyl gallate, octyl gallate and dodecyl gallate.

2. A composition according to claim 1, **characterized in that** it further comprises biochanin A or a plant extract comprising biochanin A, advantageously an extract of red clover.

3. A composition according to one of the preceding claims, **characterized in that** dihydromyricetin represents from 0.0001% to 10% by weight of the composition, advantageously from 0.001% to 2%.

4. A composition according to one of the preceding claims, **characterized in that** the zinc salt is zinc gluconate.

5. A composition according to one of the preceding claims, **characterized in that** the zinc salt represents from 0.1 to 10% by weight of the composition, advantageously from 1 to 5%.

6. A composition according to one of the preceding claims, **characterized in that** it further comprises at least one ingredient selected from:

   - an extract of *Ginkgo biloba,* advantageously an extract of *Ginkgo biloba* comprising flavonoids;
   - a meropterpene, advantageously bakuchiol;
   - a polyol, advantageously selected from xylitol, sorbitol and mannitol;
   - a keratolytic agent, advantageously selected from salicylic acid, glycolic acid, citric acid, malic acid and lactic acid;

- glycyrrhetinic acid or any of its derivatives or salts.

**7.** A composition according to one of the preceding claims, **characterized in that** it is in the form of a cream, a gel, a lotion or an emulsion, advantageously oil-in-water (O/W).

**8.** A cosmetic or dermatological composition comprising dihydromyricetin and a zinc salt for use in the prevention or treatment of acne.

**9.** A cosmetic or dermatological composition comprising dihydromyricetin and a zinc salt for its use as :

- a seboregulating agent; and/or
- a sebum thinning agent; and/or
- an agent capable of preventing the formation of atrophic acne scars; and/or
- an antimicrobial agent, especially antibacterial against *P. acnes.*

**10.** A cosmetic or dermatological composition for use according to one of claims 8 or 9, as defined in claims 1 to 7.

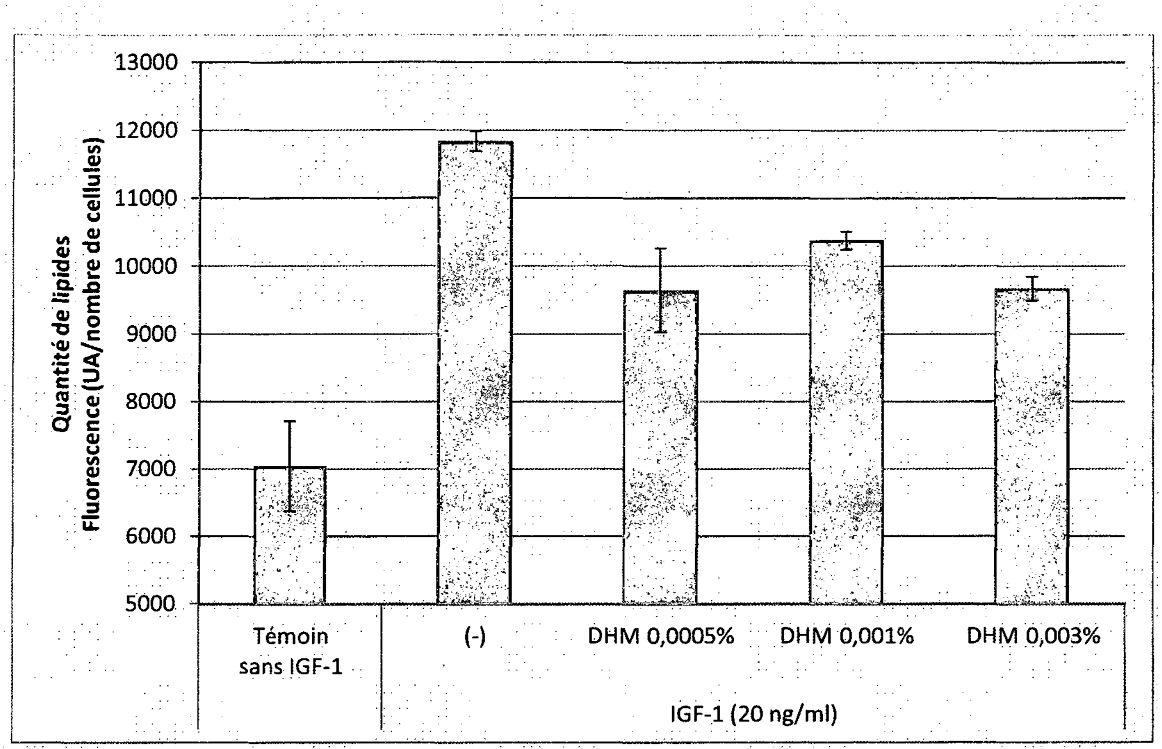


**Figure 1**

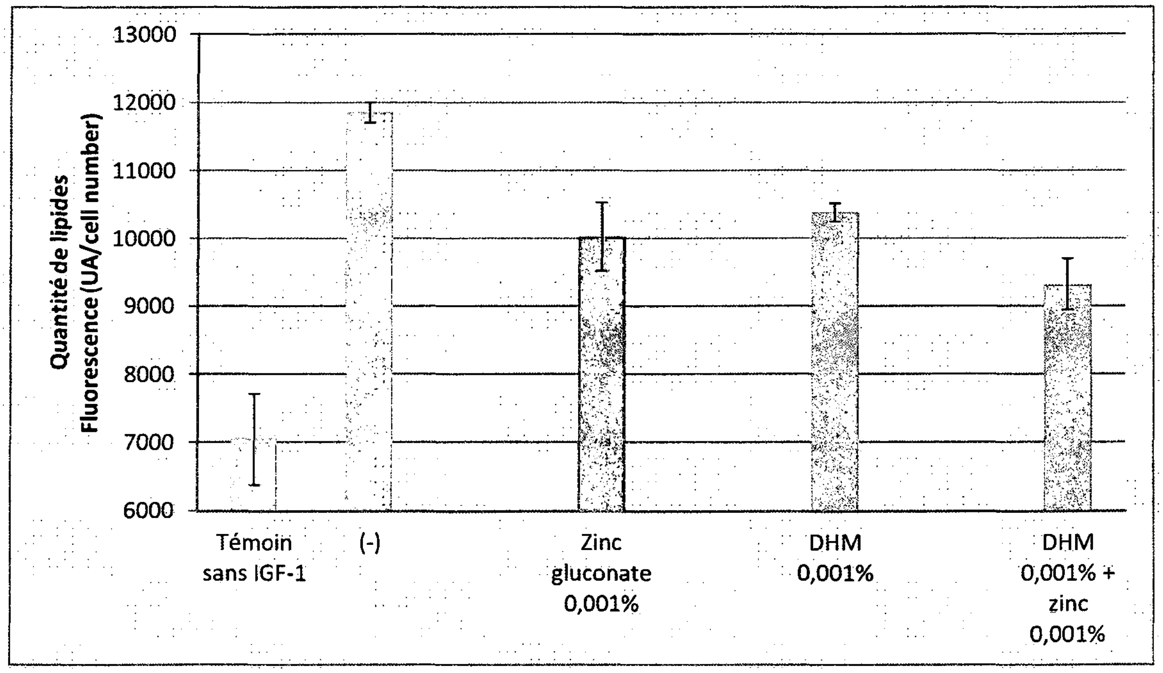


**Figure 2**

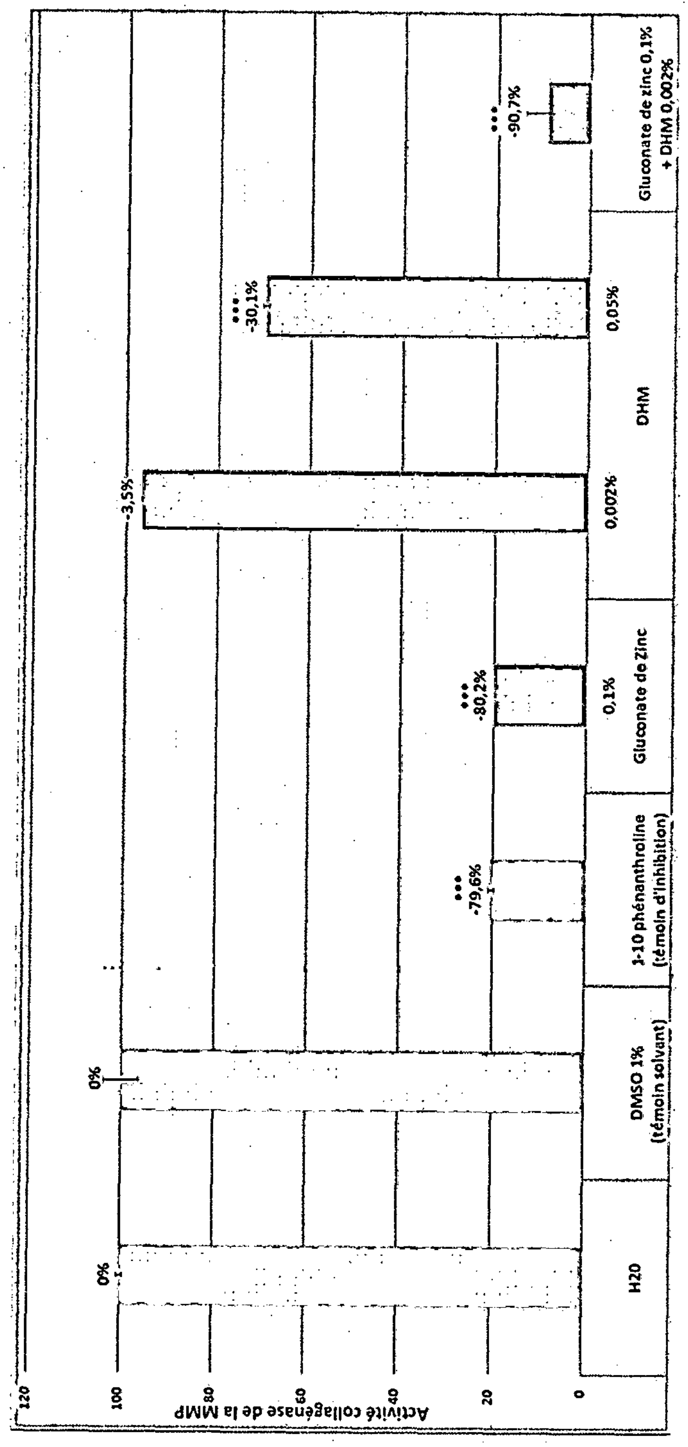
Activité collagénase de la MMP
120
100
80
60
40
20
0
0%
0%
-79,6%
-80,2%
-3,5%
-30,1%
-90,7%
H2O
DMSO 1% (témoin solvant)
1-10 phénanthroline (témoin d'inhibition)
0,1% Gluconate de Zinc
0,002%
DHM
0,05%
Gluconate de zinc 0,1% + DHM 0,002%

**Figure 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2583662 A
- CN 1483801
- JP 2000136131 B
- EP 1484086 A
- WO 2008143761 A
- WO 2008140673 A
- FR 2857266


### Littérature non-brevet citée dans la description


- *CHEMICAL ABSTRACTS,* 27200-12-0
- **BARRAULT C ; DICHAMP I ; GARNIER J ; PEDRETTI N ; JUCHAUX F ; DEGUERCY A ; AGIUS G ; BERNARD FX.** Immortalized sebocytes can spontaneously differentiate into a sebaceous-like phenotype when cultured as a 3D epithelium. *Exp Dermatol.,* Avril 2012, vol. 21 (4), 314-6
- **CAPPEL M ; MAUGER D ; THIBOUTOT D.** Correlation between serum levels of insulin-like growth factor 1, dehydroepiandrosterone sulfate, and dihydrotestosterone and acne lesion counts in adult women. *Arch Dermatol.,* 2005, vol. 141 (3), 333-8
- **CHIBA K ; SONE T ; KAWAKAMI K ; ONOUE M.** Skin roughness and wrinkle formation induced by repeated application of squalene-monohydroperoxide to the hairless mouse. *Exp Dermatol.,* Décembre 1999, vol. 8 (6), 471-9
- **CHIBA K ; YOSHIZAWA K ; MAKINO I ; KAWAKAMI K ; ONOUE M.** Comedogenicity of squalene monohydroperoxide in the skin after topical application. *J Toxicol Sci,* 2000, vol. 25, 77-83
- **CHIVOT M ; PAWIN H ; BEYLOT C ; CHOSIDOW O ; DRENO B ; FAURE M ; POLI F ; REVUZ J.** cicatrices d'acné : épidémiologie, physiopathologie, clinique, traitement. Acne scars: epidemiology, physiopathology, clinical features and treatment. *Ann Dermatol Venereol,* Octobre 2006, vol. 133, 813-824
- **DEPLEWSKI D ; ROSENFIELD RL.** Growth hormone and insulin-like growth factors have different effects on sebaceous cell growth and differentiation. *Endocrinology,* Septembre 1999, vol. 140 (9), 4089-94
- **DEPLEWSKI D ; ROSENFIELD RL.** Role of hormones in pilosebaceous unit development. *Endocr Rev.,* Août 2000, vol. 21 (4), 363-92
- **GERATS AG ; DE VLAMING P ; DOODEMAN M, AL B ; SCHRAM AW.** Genetic control of the conversion of dihydroflavonols into flavonols and anthocyanins in flowers of Petunia hybrida. *Planta,* Août 1982, vol. 155 (4), 364-8
- **HALL MJ ; MIDDLETON, RF ; WESTMACOTT D.** The fractional inhibitory concentration (FIC) index as a measure of synergy. *J. Antimicrob. Chemother.,* 1983, vol. 11 (5), 427-433
- **HORTON R ; PASUPULETTI V ; ANTONIPILLAI I.** Androgen induction of steroid 5 alpha-reductase may be mediated via insulin-like growth factor-I. *Endocrinology,* Août 1993, vol. 133 (2), 447-51
- **ISARD O ; KNOL AC ; ARIES MF ; NGUYEN JM ; KHAMMARI A ; CASTEX-RIZZI N ; DRENO B.** Propionibacterium acnes activates the IGF-1/IGF-1R system in the epidermis and induces keratinocyte proliferation. *J Invest Dermatol.,* Janvier 2011, vol. 131 (1), 59-66
- **LE LAIN R ; NICHOLLS PJ ; SMITH HJ ; MAHARLOUIE FH.** Inhibitors of human and rat testes microsomal 17beta-hydroxysteroid dehydrogenase (17beta-HSD) as potential agents for prostatic cancer. *J Enzyme Inhib.,* Janvier 2001, vol. 16 (1), 35-45
- **LENS-LISBONNE C ; CREMIEUX C ; MAILLARD C ; BALANSARD G.** Evaluation activité antibactérienne des huiles essentielles. *J. Pharm. Belg.,* 1987, vol. 42 (5), 297-302
- **OHNO S. ; MATSUMOTO N ; WATANABE M ; NAKAJIN S.** Flavonoid inhibition of overexpressed human 3beta-hydroxysteroid dehydrogenase type II. *J Steroid Biochem Mol Biol.,* Février 2004, vol. 88 (2), 175-82
- **ORFANOS CE ; ZOUBOULIS CC.** Oral retinoids in the treatment of seborrhoea and acne. *Dermatology,* 1998, vol. 196 (1), 140-7
- **OTTAVIANI M ; CAMERA E ; PICARDO M.** Lipid-médiators in acne. *Mediators Inflamm,* 25 Août 2010
- **OTTAVIANI M ; ALESTAS T ; FLORI E ; MASTROFRANCESCO A ; ZOUBOULIS CC ; PICARDO, M.** Peroxidated squalene induces the production of inflammatory mediators in HaCaT keratinocytes: a possible role in acne vulgaris. *J Invest Dermatol.,* 15 Juin 2006, vol. 126 (11), 2430-7

- **PICARDO M ; OTTAVIANI M ; CAMERA E ; MASTROFRANCESCO A.** Sebaceous gland lipids. *Dermatoendocrinol,* Mars 2009, vol. 1 (2), 68-71
- **SAINT-LÉGER D et al.** A possible role for squalene in the pathogenesis of acne. In vivo study of squalene oxides in skin surface and intra-comedonal lipids of acne patients. *Br J Dermatol,* 1986, vol. 114, 543-552
- **SHEN Y ; LINDEMEYER AK ; GONZALEZ C ; SHAO XM ; SPIGELMAN I ; OLSEN RW ; LIANG J.** Dihydromyricetin as a novel anti-alcohol intoxication medication. *J Neurosci.,* 04 Janvier 2012, vol. 32 (1), 390-401
- **STAMATIADIS D ; BULTEAU-PORTOIS MC ; MOWSZOWICZ I.** Inhibition of 5-$\alpha$-réductase activity in human skin by Zinc and azelaic acid. *British Journal of Dermatology,* 1988, vol. 119 (5), 627-632
- **THIELE JJ ; WEBER SU ; PACKER L.** Sebaceous gland secretion is a major physiologie route of vitam in E delivery to skin. *J Invest Dermatol.,* Décembre 1999, vol. 113 (6), 1006-10
- **WU S ; LIU B ; ZHANG Q ; LIU J ; ZHOU W ; WANG C ; LI M ; BAO S ; ZHU R.** Dihydromyricetin reduced Bcl-2 expression via p53 in human hepatoma HepG2 cells. *PLoS One.,* 04 Novembre 2013, vol. 8 (11), e76886